(19) Europäisches Patentamt
European Patent Office
Office européen des brevets

(11) **EP 2 484 760 B1**

(12) **EUROPEAN PATENT SPECIFICATION**

(45) Date of publication and mention
of the grant of the patent:
**14.10.2015 Bulletin 2015/42**

(51) Int Cl.:
***C12N 15/00*** (2006.01)   ***C12N 15/09*** (2006.01)
***C12Q 1/68*** (2006.01)

(21) Application number: **11772058.1**

(22) Date of filing: **21.04.2011**

(86) International application number:
**PCT/JP2011/059778**

(87) International publication number:
**WO 2011/132726 (27.10.2011 Gazette 2011/43)**

(54) **METHOD FOR PREPARING aRNA.**

VERFAHREN ZUR HERSTELLUNG VON A-RNA.

PROCÉDÉ DE PRÉPARATION D'aRNA.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2011 JP 2011013098
22.04.2010 JP 2010098639**

(43) Date of publication of application:
**08.08.2012 Bulletin 2012/32**

(73) Proprietor: **Toray Industries, Inc.
Tokyo 103-8666 (JP)**

(72) Inventors:
• **KURODA, Toshihiko**
  **Kamakura-shi, Kanagawa 248-8555 (JP)**
• **NOMURA, Osamu**
  **Kamakura-shi, Kanagawa 248-8555 (JP)**
• **NOBUMASA, Hitoshi**
  **Kamakura-shi, Kanagawa 248-8555 (JP)**

(74) Representative: **Kador & Partner
Corneliusstraße 15
80469 München (DE)**

(56) References cited:
**JP-A- 2006 501 857     JP-A- 2007 505 630
JP-A- 2009 131 262**

• **HUGHES T R ET AL: "Expression profiling using microarrays fabricated by an ink-jet oligonucleotide synthesizer", NATURE BIOTECHNOLOGY, NATURE PUBLISHING GROUP, NEW YORK, NY, US, vol. 19, no. 4, 1 April 2001 (2001-04-01), pages 342-347, XP002230790, ISSN: 1087-0156, DOI: 10.1038/86730**
• **HOEN, P. A. C. 'T. ET AL.: 'Fluorescent labelling of cRNA for microarray applications' NUCLEIC ACIDS RESEARCH vol. 31, no. 5, 2003, page E20, XP008155814**

**Description**

TECHNICAL FIELD

The present invention relates to a method for preparing aRNA to be used for gene expression analysis of an RNA sample extracted from a fixed tissue or cell(s). Further, the present invention relates to the use of a nucleotide reagent for in vitro transcription in a method for preparing amplified RNA prepared from an RNA sample extracted from a fixed tissue or cell(s).

BACKGROUND ART

In recent years, development of techniques to analyze genes contained in a vast number of samples that are stored in hospitals and research institutes in the forms of tissues and cells fixed with a fixative, such as formalin-fixed paraffin-embedded (FFPE) tissues, has been increasingly demanded. Since, especially as FFPE tissues, a vast amount of data on diseases obtained in the past have been accumulated, establishment of a technique that enables extraction, and analysis of expression, of genes extracted from FFPE tissues allows retrospective studies using tissues stored for a long period, largely contributing to future therapy and prophylaxis of diseases.

However, since degradation and fragmentation of RNA extracted from fixed tissues and fixed cells such as FFPE samples proceed under general fixation conditions and storage conditions, it has been thought that gene expression analysis is difficult with such RNA. Further, formaldehyde (formalin), which is most commonly employed as a fixative, sometimes causes RNA-RNA or RNA-protein cross-linking, or addition of formaldehyde to RNA or modification of RNA with formaldehyde. In an RNA sample in such a state, enzymatic reactions and/or chemical reactions hardly proceed in some cases, resulting in difficulty in analyzing gene expression. Therefore, a technology has been demanded which enables analysis of gene expression even with an RNA sample with extensive degradation and/or fragmentation, or with an RNA sample wherein cross-linking, addition and/or modification occurred.

Several reagent kits for amplification of RNA for the purpose of preparation of an RNA sample to be subjected to gene expression analysis, from an FFPE sample are commercially available. Particular examples of the kits include "WT-Ovation FFPE RNA Amplification System", manufactured by NuGen, which is for amplification of cDNA; and "ExpressArt TRinucleotide mRNA amplification Kit", manufactured by AmpTec, and "Paradise Plus 2 Round-Aminoallyl", manufactured by MDS Analytical Technologies, which are for amplification of aRNA. The main purpose of these kits is to obtain a large amount of an amplified product from a small amount of RNA, and therefore 2 or more times of amplification reactions are normally carried out. However, the degradation behavior of mRNA is not uniform, and therefore especially in cases where, for example, an RNA sample that was extracted from an FFPE sample and shows extensive degradation and fragmentation is to be amplified, bias due to an amplification operation very often occurs, and such bias further increases when the amplification operation is repeated a plurality of times. Thus, when the above-described kits were used to carry out gene expression analysis, the obtained result sometimes largely differed from the original abundance of RNA, so that development of a quantitative method of analysis, and reagents and kits therefor has been demanded.

As a method for amplifying an RNA sample, a method using in vitro transcription reaction is known. In this method, for the purpose of fluorescent labeling of an amplified product, a nucleotide having a functional group such as aminoallyl or biotin is sometimes used to introduce the functional group to the amplified product upon in vitro transcription reaction in the amplification operation. In this case, in order to enhance the labeling efficiency with the fluorescent dye as much as possible, the ratio of nucleotides having the functional group is increased with respect to nucleotides having no functional group. For example, an RNA sample extracted from a frozen tissue or frozen section which had not been subjected to an operation of fixation with a fixative was employed in Examples of Patent Document 1. In this example, the second round of 2 times of amplification partially used 5-(3-aminoallyl)uridine 5'-triphosphate (AA-UTP), which is a nucleotide uridine 5'-triphosphate (UTP) modified with aminoallyl group, and the ratio between AA-UTP and UTP was set to AA-UTP:UTP=3:1 (75 mol% in terms of the ratio of AA-UTP). Further, in Non-patent Document 1, the ratio between AA-UTP and UTP for enhancement of the efficiency of labeling with AA-UTP was studied, and it is described that the optimum ratio is AA-UTP:UTP=1:1 (50 mol% in terms of the ratio of AA-UTP).

PRIOR ART DOCUMENTS

Patent Document

[Patent Document 1] JP 2009-131262 A

Non-patent Document

[Non-patent Document 1] Peter A. C. 't Hoen, Floor de Kort, G. J. B. van Ommen and Johan T. den Dunnen: Fluorescent labelling of cRNA for microarray applications; Nucleic Acids Research 31(5), e20, 2003

SUMMARY OF THE INVENTION

PROBLEMS TO BE SOLVED BY THE INVENTION

As described above, performing an amplification operation of RNA a plurality of times is advantageous in view of the fact that a sufficient amount of an RNA sample for performing gene expression analysis can be obtained from a small amount of an RNA sample. However, an RNA sample extracted from a fixed tissue or cell(s) is/are degraded, fragmented, modified and/or cross-linked, and a part of the sample can be easily amplified while the other part thereof cannot be easily amplified. Therefore, as the amplification factor increases by repeating of the amplification operation, the amplification bias increases, leading to difficulty in quantitative evaluation.

On the other hand, it is said that the method of amplification of RNA by the combination of reverse transcription reaction and in vitro transcription reaction is relatively less likely to cause the amplification bias. In this method, however, in cases where a functional group is introduced for fluorescent labeling of the amplification product, a nucleotide reagent having a functional group such as the aminoallyl group needs to be used in the in vitro transcription reaction, and, if the ratio of nucleotides having a functional group is high, the amplification reaction is inhibited. Therefore, in cases where an RNA sample extracted from a fixed tissue or cell(s) showing extensive degradation, fragmentation and/or the like is used, a sufficient amount of amplification product for gene expression analysis cannot be obtained, which has been problematic. If the amplification reaction is repeated for securing a sufficient amount of amplification product, the amplification bias increases, which has also been problematic.

MEANS FOR SOLVING THE PROBLEMS

In order to solve the above problems, the present inventors intensively studied to discover that, when gene expression analysis is to be carried out with an RNA sample extracted from a fixed tissue or cell(s), inhibition of the in vitro transcription reaction can be suppressed by setting the ratio of nucleotides having an aminoallyl group in the nucleotide reagent used in the in vitro transcription reaction to within a specific range, and a sufficient amount of amplification product required for the gene expression analysis can thereby be secured. The present inventors also discovered that, by performing gene expression analysis using an amplified RNA (aRNA) sample prepared by this method, a highly quantitative analysis result can be obtained even in cases where an RNA sample extracted from a fixed tissue or cell(s) is employed, which analysis result is equivalent to a result obtained when an intact RNA sample that was extracted from a frozen tissue and hardly shows degradation or the like was employed, that is, the influence of the amplification bias can be reduced, thereby completing the present invention.

That is, the present invention is constituted by the subject-matter according to any one of claims 1 to 4.

(

EFFECT OF THE INVENTION

By using the method of the present invention for preparing aRNA, a sufficient amount of aRNA required for gene expression analysis using an RNA sample extracted from a fixed tissue or cell(s) can be obtained. Further, since, by the method of the present invention for preparing aRNA, a RNA that was less influenced by the amplification bias can be obtained, a highly quantitative gene expression analysis is possible wherein the abundance of the gene in the original RNA sample is fairly accurately reflected.

BRIEF DESCRIPTION OF THE DRAWINGS

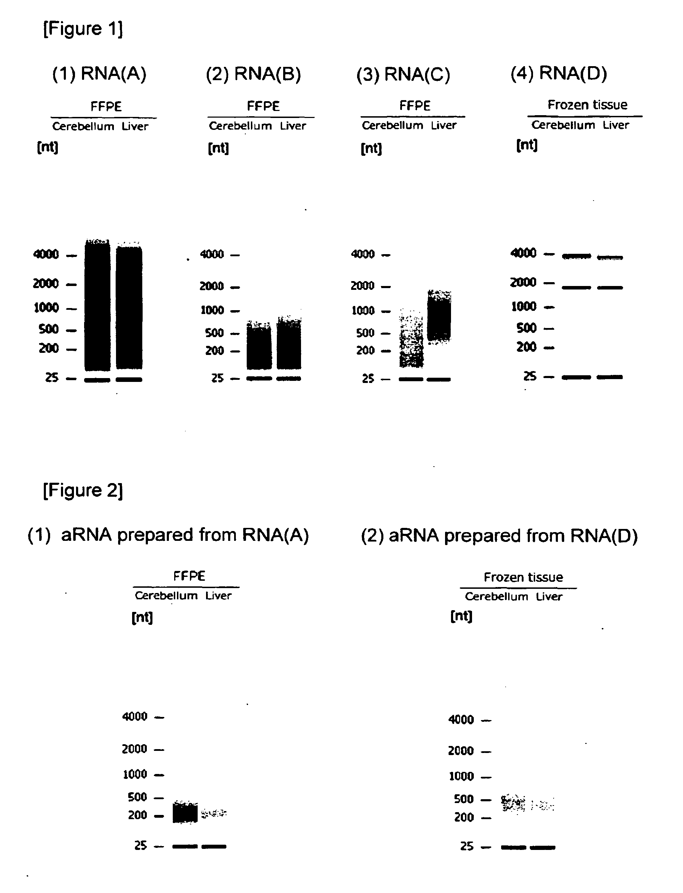

Fig. 1 shows: (1) electropherograms of RNA (A) extracted from FFPE of cerebellum and liver of mouse (A); (2) electropherograms of RNA (B) extracted from FFPE (B); (3) electropherograms of RNA (C) extracted from FFPE (C); and (4) electropherograms of RNA (D) extracted from frozen tissues of cerebellum and liver of mouse.

Fig. 2 shows (1) electropherograms of aRNA prepared from RNA (A); and (2) electropherograms of aRNA prepared from RNA (D).

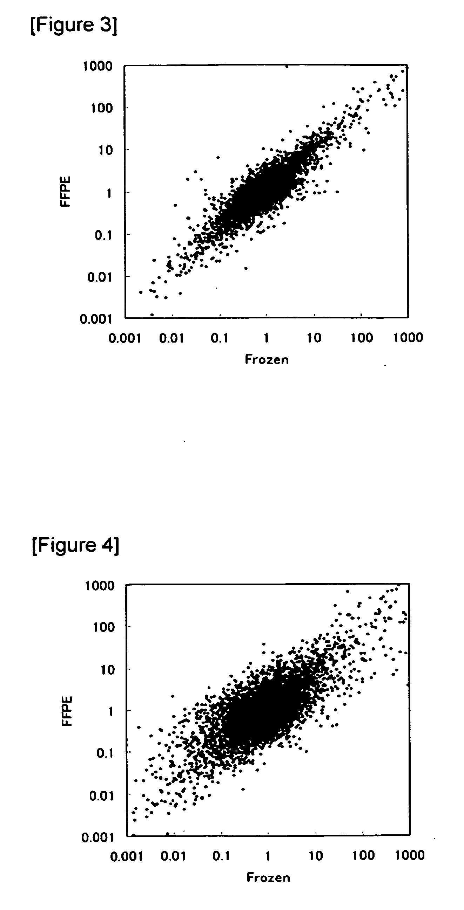

Fig. 3 is a scatter diagram of shared effective genes whose expression was confirmed in both the frozen tissue samples (D) and the FFPE samples (A) in the DNA chip analysis in Example 1, wherein the signal ratios between cerebellum and liver in the frozen tissues (cerebellum/liver) were taken along the ordinate and the signal ratios between cerebellum and liver in the FFPE samples (cerebellum/liver) were taken along the abscissa.

Fig. 4 is a scatter diagram of shared effective genes whose expression was confirmed in both the frozen tissue samples (D) and the FFPE samples (A) in the DNA chip analysis in Comparative Example 1, wherein the signal ratios between cerebellum and liver in the frozen tissues (cerebellum/liver) were taken along the ordinate and the signal ratios between cerebellum and liver in the FFPE samples (cerebellum/liver) were taken along the abscissa.

BEST MODE FOR CARRYING OUT THE INVENTION

The present invention will now be described more concretely.

The present invention relates to a method for preparing aRNA to be used for gene expression analysis with an RNA sample (which may hereinafter be referred to as "RNA sample") extracted from a fixed tissue or cell(s) (which may hereinafter be referred to as "fixed tissue or cell(s)") as defined in claim 1.

In the present invention, aRNA means amplified RNA obtained as an amplified product by subjecting the original RNA to an amplification step.

In the method of the present invention for preparing aRNA, an RNA sample extracted from a fixed tissue or cell(s) is subjected to one amplification step, to amplify messenger RNA (mRNA) in the RNA sample. In the amplification step of the present invention, RNA is amplified by the combination of reverse transcription of RNA and in vitro transcription of the resulting DNA, to obtain aRNA as an amplified product. The reverse transcription herein is carried out by performing reverse transcription reaction using single-stranded RNA, as a template, and reverse transcriptase, to obtain cDNA (complementary DNA). In general, in in vitro transcription, RNA is obtained as a transcription product from DNA as a template using RNA polymerase in vitro. By obtaining cDNA from an RNA sample by reverse transcription reaction and synthesizing double-stranded DNA, followed by performing in vitro transcription, aRNA of the antisense strand is synthesized.

Prior to the reverse transcription step, a primer from which the reverse transcription reaction is to be started is preliminarily annealed to the RNA. The primer used herein is a short fragment of nucleic acid that plays a role in supplying 3'-OH during synthesis of RNA by RNA polymerase. In the present invention, oligo-dT primers having a strand complementary to the sequence called poly-A, which exists at the 3'-end of mRNA, and random primers having normally 6 to 9 bases which can synthesize cDNA from the entire region of mRNA are suitably used. The primer used in the present invention preferably has a sequence called a promoter region. The promoter region means a region having the base sequence of a promoter to which RNA polymerase can be bound when RNA is to be amplified. Examples of the promoter which may be used in the present invention include the T7 promoter, T3 promoter and SP6 promoter. The reaction temperature for annealing of the primer is preferably 30 to 80°C, more preferably 40 to 75°C. The reaction time is preferably 5 to 120 minutes, more preferably 10 to 90 minutes.

Subsequently, in the reverse transcription step, reverse transcription reaction is performed to synthesize single-stranded cDNA (first strand cDNA) using, as a template, RNA to which the thus obtained primer was annealed, and reverse transcriptase and deoxyribonucleotides.

Reverse transcriptase is indispensable for allowing reverse transcription reaction to proceed. In the present invention, for example, enzymes derived from AMV(Avian Myeloblastosis Virus) or MMLV(Moloney Murine Leukemia Virus) may be used, and enzymes produced by modification of these enzymes by introducing a point mutation(s) and/or deletion mutation(s) to improve the heat resistance or the extensibility of bases may also be preferably used. Examples of reverse transcriptase products preferably used in the present invention include SuperScript III (Invitrogen), SuperScript II (Invitrogen), MegaScript (Ambion), PrimeScript (registered trademark, Takara Bio Inc.), ReverTra Ace (registered trademark, Toyobo Co., Ltd.) and Transcriptor Reverse Transcriptase (Roche). An RNA sample extracted from a fixed tissue or cell(s) often forms rough secondary structures due to occurrence of cross-linking and/or modification, and treatment at a high temperature is necessary to destroy such structures in some cases, so that SuperScript III, which has a high activity even at a high temperature, is especially preferably employed.

The nucleotides used in the reverse transcription step are 4 types of deoxynucleoside triphosphate (dNTP), that is, deoxyadenosine triphosphate (dATP), deoxyguanosine triphosphate (dGTP), deoxythymidine triphosphate (dTTP) and deoxycytidine triphosphate (dCTP). These are used as sources of cDNA synthesized in the reverse transcription reaction.

In the reverse transcription step, in order to avoid decrease in the activity due to oxidation of reverse transcriptase, a reducing agent may be added to the reaction solution. Examples of the reducing agent which may be suitably used include dithiothreitol (DTT). Further, for protecting the RNA as a template, an RNase inhibitor is preferably added to the reaction solution. Further, in order to adjust the pH of the reaction solution to a pH appropriate for reverse transcription reaction, which is between 7 and 9, a buffer may be used. Examples of the buffer which is preferably used include Tris-HCl buffer and phosphate buffer. Further, the reaction solution preferably contains a potassium salt and/or a magnesium salt.

The temperature of the reverse transcription reaction is preferably 25 to 60°C, more preferably 30 to 50°C, still more preferably 35 to 45°C. The time of the reverse transcription reaction is preferably 30 to 180 minutes, more preferably 60 to 150 minutes. After the reverse transcription reaction, the reaction is stopped by, for example, transferring the reaction solution onto ice for rapid cooling.

After completion of the reverse transcription reaction, a part of the RNA used as the template for the reverse transcription reaction is degraded with ribonuclease (RNase). RNase H is suitable as the reagent to be used in this treatment. By adding RNase H to the reaction solution after the completion of reverse transcription reaction and allowing thermal reaction to proceed, RNA can be degraded. The temperature of this degradation reaction is preferably 10 to 50°C, more preferably 15 to 40°C. Further, after the degradation reaction, for example, a step of heating at 90 to 100°C for 1 to 10 minutes may be included as a step of deactivation of the reverse transcriptase.

Thereafter, the single-stranded cDNA (first strand cDNA) obtained by the above-described reverse transcription step is used as a template to synthesize DNA complementary thereto (second strand cDNA). In terms of the primer, a part of RNA which was not degraded in the reverse transcription step may be used as the primer, or an exogenous primer may be annealed to be used as the primer, as required. By adding dNTPs and DNA polymerase, as an enzyme, to cDNA as a template, the reaction starts, and the first strand cDNA is used as the template to synthesize the second strand cDNA. As a result of the synthesis reaction of the second strand cDNA, double-stranded DNA having a promoter sequence is synthesized.

The double-stranded DNA having a promoter sequence, which was obtained by the second strand cDNA synthesis reaction, is preferably purified in order to remove salts and impurities that inhibit reaction in the subsequent in vitro transcription step. The purification may be carried out by, for example, ethanol precipitation, or a method using a commercially available spin column for DNA purification, which method is known to those skilled in the art.

Using the purified double-stranded DNA having a promoter sequence, a step of in vitro transcription (IVT) is carried out to amplify RNA, and the aRNA of interest is thereby obtained. In the in vitro transcription step, aRNA is obtained by performing in vitro transcription reaction by adding a nucleotide regent containing adenosine 5'-triphosphate (ATP), guanosine 5'-triphosphate (GTP), cytidine 5'-triphosphate (CTP), uridine 5'-triphosphate (UTP) and 5-(3-aminoallyl)uridine 5'-triphosphate (AA-UTP), and RNA polymerase that recognizes the promoter sequence, to the double-stranded DNA having a promoter sequence. Here, the reaction temperature of the in vitro transcription is preferably 30 to 50°C, more preferably 35 to 45°C. The reaction time of the in vitro transcription is preferably 1 to 20 hours, more preferably 2 to 16 hours. In the in vitro transcription, an RNase inhibitor (e.g., dithiothreitol), reducing agent and/or the like may be used as appropriate.

In the nucleotide reagent used in the in vitro transcription step, 5-(3-aminoallyl)uridine 5'-triphosphate (AA-UTP) prepared by modifying uridine 5'-triphosphate (UTP) with the aminoallyl group is included as a nucleotide having an aminoallyl group for fluorescent labeling of the prepared aRNA. In the present invention, the ratio of the content of AA-UTP with respect to the total of UTP and AA-UTP, that is, the ratio calculated according to "molar amount of AA-UTP / (molar amount of UTP + molar amount of AA-UTP)" is not less than 5 mol% and less than 25 mol%. The lower limit of the ratio of the content of AA-UTP is preferably not less than 6 mol%, more preferably not less than 8 mol%, still more preferably not less than 10 mol%. The upper limit of the ratio of the content of AA-UTP is preferably not more than 24 mol%, more preferably not more than 23 mol%. Further, the range of the ratio of the content of AA-UTP is preferably not less than 6 mol% and not more than 24 mol%, more preferably not less than 10 mol% and not more than 23 mol%. In cases where the ratio of the content of AA-UTP is not less than 25 mol%, the progress of in vitro transcription reaction is inhibited, which may result in an insufficient amount of aRNA for gene expression analysis. Further, in cases where the ratio of AA-UTP is less than 5 mol%, the amount of the fluorescent label with respect to the amount of nucleotides is small and hence the detection sensitivity is low, and, as a result, the information of gene expression obtained in the gene expression analysis may be poor.

After the in vitro transcription reaction, in order to remove the double-stranded DNA remaining in the aRNA solution, the DNA is degraded using DNase. The amount of the DNase to be added is not restricted as long as the remaining DNA can be sufficiently degraded. For example, in cases where RNase-Free DNase I (1 MBU/μL) (MBU: Molecular Biology Unit) (Epicentre) is used as the DNase, use of 1 μL thereof is enough for degrading 1 μg of DNA at 37°C for 10 minutes. Usually, in the DNase treatment, the DNA can be degraded by incubating the solution at 15 to 40°C for 10 to 60 minutes.

Thereafter, the thus prepared aRNA is preferably purified before being subjected to gene expression analysis. Preferred examples of the method of purification of the aRNA include a method wherein a spin column having a silica membrane is used to allow adsorption of the aRNA to the membrane and then to remove unnecessary substances by washing, followed by elution of the adsorbed aRNA. The washing solution to be used in this case preferably contains a guanidium salt and an alcohol. Preferred examples of the eluent include nuclease-free water and TE buffer. The eluent may be used either at room temperature or after being heated.

The fluorescent labeling of the aRNA obtained by the preparation method of the present invention may be carried out either before or after the hybridization reaction. The aminoallyl groups introduced by the present invention can be simply coupled with a fluorescent dye having, at its terminus, an N-hydroxysuccinimide (NHS) group. Examples of the fluorescent dye which can be used herein include, but are not limited to, Cy3, Cy5, Hyper5 (GE Healthcare), Alexa Fluor (registered trademark) series (Molecular Probes), Oyster series (Denovo Biolabels), DyLight series (Pierce), Fluolid-W series (IST), HyLite Fluor series (Anaspec) and MegaStokes Dye (Dyomics).

The fixed tissue or cell(s) to which the method of the present invention for preparing aRNA is to be applied is/are fixed. Examples of the fixative used for fixing the tissue or cell(s) include formaldehyde solutions; paraformaldehyde solutions; solutions containing an alcohol such as ethanol, or containing acetone or chloroform; solutions containing an acid such as picric acid or potassium dichromate; solutions containing a metal such as zinc acetate, zinc chloride or zinc sulfate; among which a formaldehyde solution or paraformaldehyde solution is preferably used. Preferably, the formaldehyde solution to be employed may be prepared by diluting commercially available formalin (formaldehyde concentration, 37%) with water; by diluting a formalin solution with water and adjusting the pH of the resulting dilution to neutral by addition of calcium carbonate or magnesium carbonate; or by dilution with phosphate buffer to adjust the pH to neutral. The formaldehyde content in the formaldehyde solution is preferably 1 to 30%, more preferably 2 to 20%.

The tissue or cell(s) may be embedded in paraffin. For example, a formalin-fixed paraffin-embedded (FFPE) tissue or cell(s) may be preferably used. The fixed tissue or cell(s) may be embedded in paraffin by a common method which is known to those skilled in the art. For example, the fixed tissue or cell(s) is subjected to substitution with alcohol to dehydrate the tissue or cell(s), followed by substitution with xylene, benzene or the like. In a mold into which paraffin melt by heating was poured, the tissue or cell(s) is/are placed and embedded, to provide a paraffin block. In cases where an RNA sample is extracted from a paraffin embedded tissue or cell(s), the tissue or cell(s) may be sliced using a microtome such as a rotary microtome or sliding microtome before use. The thickness of each thin section is not restricted, and preferably 1 to 100 $\mu$m, more preferably 2 to 50 $\mu$m. The embedding of the fixed tissue or cell(s) may also be carried out using, instead of paraffin, OCT (Optical Cutting Temperature) compound, which is mainly used for preparation of frozen tissue sections.

The paraffin-embedded fixed tissue or cell(s) may be sliced into a thickness of 1 to 20 $\mu$m and attached to a slide glass, thereby cutting out only a part of the tissue or cell(s) to be employed. This method is preferred since the analysis can be carried out for only the tissue or cell(s) of interest which is/are intended to be the subject of gene expression analysis. Since, especially in the case of a formalin-fixed paraffin-embedded (FFPE) tissue or cell(s), the tissue or cell(s) is/are fixed, the tissue or cell(s) can be said to be a sample suitable for the above-described operation. Examples of the method to cut out the tissue or cell(s) include laser-capture microdissection (LCM). In cases where a tissue or cell(s) is/are collected by LCM, the amount of the tissue or cell(s) to be collected is usually small, so that the amount of RNA which can be extracted is small. Therefore, the analysis can be preferably carried out with a small amount of RNA.

Especially in cases where only the tissue or cell(s) of interest which is/are intended to be the subject of gene expression analysis is/are collected as described above, the fixed tissue or cell(s) may be stained such that the shape, properties and/or the like of the tissue or cell(s) can be identified. Preferred examples of the staining solution to be employed include cresyl violet, toluidine blue, hematoxylin and nuclear fast red.

Examples of the fixed tissue or cell(s) to which the method of the present invention for preparing aRNA is to be applied include organs and tissues which were surgically removed; organs and tissues collected by needle biopsy for body tissue examination and the like; organs and tissues of various experimental animals; primary cultured cells; and cell lines.

As a method for extracting RNA from the fixed tissue or cell(s), a known method using a reagent for deparaffinization (e.g., xylene or limonene), alcohol (e.g., ethanol), enzyme for digesting proteins of a tissue or cell(s) (e.g. proteinase K) and a means of purification of extracted RNA may be applied. Further, examples of commercially available products which may be preferably employed include RNA extraction kits for formalin-fixed paraffin-embedded tissues, such as "RecoverAll$^{(TM)}$ Total Nucleic Acid Isolation Kit for FFPE" (manufactured by Ambion), "RNeasy FFPE Kit" (manufactured by Qiagen), "ISOGEN PB Kit" (manufactured by Nippon Gene Co., Ltd.), "FFPE RNA Purification Kit" (manufactured by Norgen), "PureLink$^{(TM)}$ FFPE RNA Isolation Kit" (manufactured by Invitrogen) and "High Pure FFPE RNA Micro" (manufactured by Roche Applied Science).

In the method of the present invention for preparing aRNA, the amount of RNA to be subjected to the RNA amplification step is preferably 10 to 2000 ng, more preferably 50 to 1500 ng in terms of the total RNA extracted as described above.

Further, in the method of the present invention for preparing aRNA, the number of times of the amplification step to be carried out is one.

As the RNA sample extracted from a fixed tissue or cell(s) to be used in the method of the present invention for preparing aRNA, RNA maintaining a certain level of quality and having only relatively low levels of degradation, fragmentation, modification and/or cross-linking as described above is preferably employed in view of obtaining more useful results in gene expression analysis. For example, in the size distribution of the extracted RNA sample observed by electrophoresis using Bioanalyzer 2100 (Agilent Technologies), the ratio (%) of RNA molecules having 1000 to 4000 nucleotides, A, relative to the ratio (%) of RNA molecules having more than 4000 nucleotides, B, is preferably A ≥ B. In cases where the RNA sample satisfies this condition, the ratio of modified and/or cross-linked RNA molecules is relatively small and hence useful analysis results are more likely to be obtained therewith. Further, for example, the larger the ratio (%) of RNA molecules having 1000 to 4000 nucleotides, A, in the size distribution of the extracted RNA sample observed by electrophoresis using Bioanalyzer 2100, the smaller the ratio of modified, cross-linked, degraded and/or fragmented RNA molecules in the RNA. Therefore, the RNA sample can be said to be in a state closer to an intact RNA sample, and useful analysis results are more likely to be obtained therewith. More particularly, in an RNA sample which may be employed, the ratio (%) of RNA molecules having 1000 to 4000 nucleotides, A, is preferably not less than 15%, more preferably not less than 20%, still more preferably not less than 25%.

In order to measure the size distribution of the above RNA sample, the relative abundance of RNA molecules in a specific molecular weight range can be calculated by, for example, performing electrophoresis of the RNA using RNA6000 Nano chip or RNA6000 Pico chip (both of which are manufactured by Agilent Technologies) according to the method described in the attached instruction, followed by analyzing the obtained electrophoretic pattern using the Smear analysis function included in the dedicated software. In cases where undegraded RNA (intact RNA) is analyzed in this method, ribosomal RNA which is said to have about 4700 nucleotides may appear as a band having a peak at a molecular weight corresponding to that of RNA having a little less than 4000 nucleotides. It is thought that this is due to the fact that each 28S molecule contains many double-stranded regions and hence has a compact structure, resulting in a migration rate faster than that expected from its actual molecular weight. A similar description can be found in FAQ shown in the homepage of Agilent Technologies. Therefore, in the present method, 28S ribosomal RNA is regarded as being substantially within the range of 1000 to 4000 nucleotides in electrophoresis. Further, since the molecular weight of 18S corresponds to that of 1874 nucleotides and the band of 18S appears within the range of 1000 to 4000 nucleotides in electrophoresis, 18S is of course included within the range.

The method for analyzing gene expression with an RNA sample employs aRNA prepared by the preparation method of the present invention described above. This analysis can be carried out by binding reaction of fluorescently labeled aRNA with a selective binding substance which can directly or indirectly, and selectively bind to the RNA of interest whose expression is to be analyzed. For example, a microarray having a surface on which a selective binding substance(s) is/are immobilized is preferably used. Examples of the selective binding substance include nucleic acids and other antigenic compounds. Examples of the nucleic acids herein include deoxyribonucleic acid (DNA), ribonucleic acid (RNA), peptide nucleic acid (PNA), complementary DNA (cDNA) and complementary RNA (cRNA). The selective binding substance is preferably nucleic acid, more preferably an oligomer of DNA or RNA having a part of the sequence complementary to the sequence of the gene to be analyzed in the aRNA, still more preferably a DNA oligomer, which binds to the RNA more strongly. The length of the DNA oligomer is preferably 20 to 100 mer, more preferably 40 to 80 mer. The selective binding substance may be one which is commercially available, synthesized, or prepared from a natural source such as a body tissue or cell(s). Further, in cases where a microarray is used, all the selective binding substances immobilized on the array preferably have sequences different from each other.

In the analysis of gene expression with an RNA sample, aRNA is preferably fragmented in order to increase the binding capacity between the selective binding substance and the RNA to be analyzed. The size of the fragmented aRNA is preferably 100 to 200 nucleotides on average.

In terms of the method of binding of the aRNA with the selective binding substance immobilized on the microarray, the binding is preferably carried out by hybridization reaction especially in cases where the selective binding substance is nucleic acid. The reaction solution used in the hybridization preferably contains a buffering component (e.g., phosphate buffer or SSC) for stabilization of the pH of the solution; formamide, which plays a role in decreasing the melting temperature (Tm); surfactant (e.g., lauryl sulfate or lauroyl sarcosinate); and/or blocking agent (Denhardt's reagent, fragmented salmon sperm DNA, Cot1 DNA or tRNA) for preventing non-specific binding. In cases where the hybridization reaction is performed by applying a reaction solution containing fluorescently labeled aRNA to a microarray, the reaction is preferably carried out under conditions appropriate for the microarray used. In terms of the method for detecting the amount of the nucleic acid of aRNA bound to the selective binding substance immobilized on the microarray, the amount can be detected/measured by reading the intensity of the fluorescence with which the nucleic acid was labeled, using a fluorescence scanning apparatus for microarray, for example, GenePix (registered trademark) 4000B (Molecular Devices); ScanArray (registered trademark) Lite or ScanArray (registered trademark) Express (these two are manufactured by Parkin Elmer); CRBIO IIe (registered trademark) (Hitachi Software Engineering); or 3D-Gene (registered trademark) Scanner (Toray Industries, Inc.). Based on the detected fluorescence intensity, the expression level of each gene is quantified.

As the microarray used in the microarray analysis of aRNA, a microarray prepared by binding a selective binding substance to a substrate composed of inorganic material(s) such as glass, ceramics and/or silicone; metal(s) such as stainless steel and/or gold (gilding); and/or macromolecular material(s) such as polyethylene terephthalate, polymethylmethacrylate (PMMA) and/or silicone rubber may be used. Further, a commercially available microarray may also be used.

A preferred microarray may have an irregular portion(s) on the surface of the substrate, and may further have a cover over a top face(s) of a protruded portion(s). In such a case, the cover preferably has one or more penetrating holes communicating with a void(s), for injection of a liquid. A microarray wherein microparticles are encapsulated in the void(s) between the microarray and the cover is especially preferred since, after application of a sample solution to the void(s), vibration, shaking, rotation, magnetic force, gravity and/or the like may be applied to the microarray to move the encapsulated microparticles, thereby stirring the sample solution, which results in promotion of the hybridization reaction. The material of the microparticles herein is not restricted, and preferred examples thereof include glass, ceramics (e.g., yttria-stabilized zirconia), metals (e.g., stainless steel), polymers (e.g., nylons and polystyrenes) and magnetic bodies.

In cases where aRNA is analyzed with a microarray, the amplification factor of the aRNA is preferably 2 to 20. In cases where an amplification factor of only less than 2 can be obtained with an RNA, the RNA may be extensively degraded and fragmented and hence RNA strands are very short, or the amplification reaction may be inhibited by cross-linking between RNA molecules or between RNA and protein or by addition/modification due to binding of a substance derived from a fixative such as formaldehyde to RNA during fixation. Therefore, in such cases, amplification of the RNA may be impossible or the amplification reaction may be insufficient, and, as a result, accurate microarray analysis may be impossible. Further, in cases where the amplification product was obtained with an amplification factor higher than 20, the amplification bias may remarkably appear as an influence of the difference in the level of degradation and/or the like.

Examples of a method for adjusting the amplification factor to within 2 to 20 include, but are not limited to, optimization of the composition of the reagent by increasing and/or decreasing the concentrations of the enzyme, primer, substrate and/or the like used in the amplification step of the RNA. Another method is to control the reaction time. For example, in cases where an RNA sample extracted from a fixed tissue or cell(s) is amplified using a reagent that leads to a relatively high amplification factor, the reaction time may be set shorter than the prescribed length of time.

The amplification factor upon amplification of an RNA sample by the amplification step of the present invention may be calculated as the ratio between the weights of RNA measured before and after the amplification. More particularly, based on the fluorescence intensity measured with a spectrophotometer using a cell having an optical path length of 10 mm at a wavelength of 260 nm and the solution volume, the weights of RNA in the solution before and after the amplification may be determined according to Equation A below, and the amplification factor may then be calculated according to Equation B below.

$$\text{Equation A: RNA weight} = (\text{fluorescence intensity at 260 nm}) \times 40 \ (\text{ng}/\ \mu\text{L})$$

$$\times \text{ solution volume } (\mu\text{L})$$

$$\text{Equation B: Amplification factor} = (\text{RNA weigh after amplification}) / (\text{RNA}$$

$$\text{weight before amplification})$$

Examples of the standard for judging whether or not aRNA that produces results similar to those obtained with an intact RNA showing no degradation could be prepared from an RNA sample extracted from a fixed tissue or cell(s) by the method of the present invention for preparing aRNA include confirmation of the size of aRNA. In cases where the RNA sample is extensively degraded or the RNA has modification and/or cross-linking, cDNA chains obtained by the reverse transcription reaction are short, and, as a result, the obtained aRNA chains are also short. Such an RNA sample tends to show a considerably low amplification factor, and, in cases where gene expression analysis is carried out with such an RNA sample, the number of effective spots is smaller than in cases where intact RNA having no degradation was employed, and the correlation between the both tends to be worse. The size of aRNA can be confirmed by using electrophoresis, liquid chromatography or the like. For example, the size is preferably measured using a chip electrophoresis system such as "Agilent 2100 bioanalyzer" (Agilent Technologies). When the size distribution of aRNA as the amplification product was investigated by this method and the size (nucleotide length) corresponding to the peak of the distribution was confirmed, in cases where the size is not less than 200 nucleotides ([nt]), it can be said that there is a high possibility that results of gene expression analysis show high correlation with results obtained using intact RNA showing no degradation. On the other hand, in cases where the peak of distribution corresponded to less than 200 [nt], it is suggested that the quality of the original RNA sample was poor, and there is a high possibility that plausible results cannot be obtained in gene expression analysis.

EXAMPLES

The present invention will now be described by way of Examples below in more detail.

Reference Example 1 Quantification of AA-UTP and UTP

The contents of 5-(3-aminoallyl)uridine 5'-triphosphate (AA-UTP) and UTP were quantified as follows by LC/MS analysis.
Using standard products of AA-UTP (Ambion) and UTP (Roche), a concentration dilution series was prepared with each of these. LC/MS/MS measurement was carried out using Prominence HE-UV (Shimadzu Corporation) as an HPLC apparatus, LCMS-IT-TOF (Shimadzu Corporation) as a mass spectrometer, and CapcelPak C18 AQ (Shiseido Co., Ltd.) as a column. In the mass spectrum at the retention time for the peak derived from each of UTP and AA-UTP detected in LC, the area of a peak having a value close to the theoretical m/z value of the ion derived from UTP, 482.9607, or the ion derived from AA-UTP, 538.0029, respectively, was determined to prepare a calibration curve. Thereafter, by drawing a mass chromatogram in the same manner using a sample to be measured and determining the areas of the peaks, the UTP concentration and the AA-UTP concentration in the sample were quantified based on the calibration curves.

Reference Example 2 Extraction of RNA from FFPE Sample and Confirmation of Quality

(1) RNA Samples (A) to (C)

As fixed paraffin-embedded (FFPE) block samples of liver and cerebellum of mice (7 weeks old, male, Slc:ICR), the following FFPE (A) to (C) were prepared.
FFPE (A): Sample prepared by fixation with 10% neutral buffered formalin solution for 2 days and embedding in paraffin, followed by storage at room temperature for about 6 months.
FFPE (B): Sample prepared by fixation with 10% neutral buffered formalin solution for 2 days and embedding in paraffin, followed by storage at room temperature for about 1 year.
FFPE (C): Sample prepared by fixation with 4% paraformaldehyde solution for 1 day and embedding in paraffin, followed by storage at room temperature for about 6 months.
From the paraffin blocks of the above FFPE (A) to (C) (liver and cerebellum), 2 thin sections of liver and 10 thin sections of cerebellum each having a thickness of 10 $\mu$m were collected using a microtome, and each of these was placed in a 1.5-mL tube. To each tube, 1 mL of xylene was added, and the resultant was stirred to dissolve paraffin. After centrifugation at 16,000×g for 5 minutes, xylene was removed with a pipette. Subsequently, 1 mL of ethanol was added to the tube, and the resultant was stirred, followed by 2 minutes of centrifugation at 16,000×g for 2 minutes and sufficient removal of ethanol with a pipette. This operation was repeated twice. The lid of each tube was left open for about 10 minutes for air drying, to remove ethanol contained in each tissue. To each tube, 100 $\mu$L of a proteinase K solution (500 $\mu$g/mL) was added, and each tissue was suspended therein, followed by leaving the suspension to stand at 37°C for 16 hours. Centrifugation was carried out at 16,000×g for 2 minutes to remove the residue, and RNA was purified using a silica column.
Thus, RNA (A), RNA (B) and RNA (C) were obtained from FFPE (A), FFPE (B) and FFPE (C), respectively.
The purity of each of RNAs (A) to (C) (the ratio of the intensity at a wavelength of 260 nm with respect to the intensity at a wavelength of 280 nm) was measured with a spectrophotometer (Nano Drop), and, as a result, as respectively shown in Table 1, it was revealed that the purities of these RNA samples are high. Further, as a result of electrophoresis using Agilent 2100 Bioanalyzer RNA6000 Nano chip (Agilent Technologies), the degradation behavior of RNA varied among the samples, as shown in Fig. 1 (1) to (3).
The qualities of the above-extracted RNAs (A) to (C) were evaluated as follows. Using the Smear analysis function included in the dedicated software for Agilent 2100 Bioanalyzer, the ratio (%) of RNA molecules having 1000 to 4000 nucleotides, A, and the ratio (%) of RNA molecules having more than 4000 nucleotides, B, were calculated for the electrophoretic pattern of each RNA sample, and the ratio between A and B, B/A, was calculated and used as an index of the quality. Here, it can be said that a higher A indicates a higher quality, and B/A of not more than 1 indicates a relatively high quality of RNA. The results are shown in Table 1. The qualities of RNAs (A) to (C) were excellent.

[Table 1]

| Tissue sample | FFPE (A) | | FFPE (B) | | FFPE (C) | |
|---|---|---|---|---|---|---|
| | Cerebellum | Liver | Cerebellum | Liver | Cerebellum | Liver |
| RNA sample | RNA (A) | | RNA (B) | | RNA (C) | |
| Purity (260 nm/280 nm) | 2.05 | 1.99 | 1.99 | 1.98 | 2.02 | 1.98 |
| Ratio of 1000-4000 [nt], A (%) | 37 | 41 | 21 | 20 | 26 | 35 |
| Ratio of more than 4000 [nt], B (%) | 1 | 1 | 1 | 2 | 2 | 1 |
| B/A | 0.03 | 0.02 | 0.05 | 0.10 | 0.08 | 0.03 |

(2) RNA Sample (D) as Control

As a control RNA sample, RNA (D) was extracted from each of freshly frozen tissues of liver and cerebellum of mice (7 weeks old, male, Slc:ICR). In the same manner as in (1) described above, the purity and the quality of each RNA sample was evaluated, and the results are shown in Table 2. Further, electrophoresis was carried out in the same manner as in (1) described above, and, as a result, as shown in Fig. 1 (4), the samples from both liver and cerebellum had excellent qualities as can be seen by the fact that the bands for 18S and 28S ribosomal RNAs could be clearly confirmed.

[Table 2]

| Tissue sample | Frozen | |
|---|---|---|
| | Cerebellum | Liver |
| RNA sample | RNA (D) | |
| Purity (260 nm/280 nm) | 2.08 | 2.07 |
| Ratio of 1000-4000 [nt], A (%) | 89 | 87 |
| Ratio of more than 4000 [nt], B (%) | 2 | 3 |
| B/A | 0.02 | 0.03 |

(3) RNA Samples (A') to (C')

Sections having a thickness of 10 $\mu$m were prepared from each of the paraffin blocks of FFPE (A) to (C), and attached to a slide glass dedicated to laser microdissection. After deparaffinization, the sections were stained with cresyl violet according to a conventional method, and a part of each tissue was collected using a laser microdissection (LMD) apparatus (manufactured by Leica). The area of collection in this operation was about 10,000,000 $\mu$m$^2$. RNA was extracted in the same manner as in (1) described above except that the amount of the Proteinase K solution was 25 $\mu$L, to obtain RNA (A'), RNA (B') and RNA (C'), respectively, as RNA samples. In the same manner as in the above (1), the purities and the qualities of these RNA samples were measured. As shown in Table 3, the purities and the qualities were almost equivalent to those of the corresponding RNAs (A) to (C). Further, the results obtained with RNAs (A') to (C') by performing electrophoresis using Agilent 2100 Bioanalyzer RNA6000 Pico chip (Agilent Technologies) (not shown) were almost the same as the results obtained with RNAs (A) to (C) ((1) to (3) in Fig. 1), respectively.

[Table 3]

| Tissue sample | FFPE (A') | | FFPE (B') | | FFPE (C') | |
|---|---|---|---|---|---|---|
| | Cerebellum | Liver | Cerebellum | Liver | Cerebellum | Liver |
| RNA sample | RNA (A') | | RNA (B') | | RNA (C') | |
| Purity (260 nm/280 nm) | 2.01 | 1.97 | 2.00 | 1.96 | 2.01 | 1.99 |
| Ratio of 1000-4000 [nt], A (%) | 36 | 39 | 20 | 19 | 25 | 33 |
| Ratio of more than 4000 (nt), B (%) | 1 | 1 | 1 | 1 | 2 | 1 |

(continued)

| Tissue sample | FFPE (A') | | FFPE (B') | | FFPE (C') | |
|---|---|---|---|---|---|---|
| | Cerebellum | Liver | Cerebellum | Liver | Cerebellum | Liver |
| B/A | 0.03 | 0.03 | 0.05 | 0.06 | 0.04 | 0.03 |

Example 1

(1) Preparation of aRNA (Step of Amplification of Extracted RNA)

In each PCR tube, 1 $\mu$g of RNA (A) or RNA (D) from mouse liver or cerebellum extracted in Reference Example 2 was placed, and a primer containing a T7 promoter region was added thereto, followed by allowing the reaction to proceed at 70°C for 1 hour, to perform annealing. Reverse transcriptase (SuperScript III (Invitrogen)), dNTPs (dATP, dGTP, dTTP and dCTP), RNase inhibitor and DTT were added to the tube, and the resulting mixture was sufficiently mixed, followed by allowing the reaction to proceed at 42°C for 90 minutes to synthesize the first strand cDNA. By rapidly cooling the reaction solution on ice, the reaction was stopped. RNase H was added to the reaction solution and the resulting mixture was mixed, followed by incubation at 37°C for 30 minutes and further at 95°C for 5 minutes. Subsequently, DNA polymerase and dNTPs were added thereto to synthesize the second strand cDNA, which is complementary to the first strand cDNA. The synthesized cDNA was purified through a silica gel-based column, and a nucleotide reagent (a mixture of ATP, GTP, CTP, UTP and AA-UTP), RNase inhibitor, DTT and RNA polymerase that recognizes the T7 promoter sequence were added thereto, followed by gentle mixing of the resulting mixture and allowing the reaction to proceed at 42°C for 10 hours, to perform in vitro transcription reaction using T7 RNA polymerase and hence to perform amplification reaction of aRNA to which aminoallyl groups were introduced. By the method described in Reference Example 1, the ratio of AA-UTP contained in the nucleotide reagent used in the in vitro transcription reaction, "molar amount of AA-UTP / (molar amount of UTP + molar amount of AA-UTP)", was quantified, and the ratio was found to be 16%. The amplified aRNA was purified using a silica gel-based column, and the yield was determined with a spectrophotometer (manufactured by Nano Drop), and found to be: liver FFPE, 3.5 $\mu$g; cerebellum FFPE, 4.8 $\mu$g; liver frozen tissue, 10.9 $\mu$g; and cerebellum frozen tissue, 12.7 $\mu$g (the amplification factors were 3.5, 4.8, 10.9 and 12.7, respectively) (Table 4). As a result of confirmation of the sizes of the aRNAs by electrophoresis using Agilent 2100 Bioanalyzer (Agilent Technologies), each aRNA obtained from RNA (A) derived from FFPE (A) showed a distribution having a peak between 200 and 300 [nt] for both liver and cerebellum as shown in Fig. 2(1), and each aRNA obtained from RNA (D) derived from frozen tissues showed a distribution having a peak at about 500 [nt] for both liver and cerebellum as shown in Fig. 2(2).

(2) Fluorescent Labeling and Fragmentation of aRNA

The solution of each amplified aRNA was concentrated using a centrifugal concentrator (MV-100, (Tomy Seiko Co., Ltd.)) to about 1 $\mu$L. To the resulting concentrate, 2.5 $\mu$L of Sodium Bicarbonate Buffer, which is attached to the "3D-Gene (registered trademark) Hybridization Buffer" kit (Toray Industries, Inc.), was added, and the resulting mixture was stirred by pipetting, followed by adding 2.5 $\mu$L of Cy5-NHS (GE Healthcare) dissolved in DMSO thereto, stirring the resulting mixture by pipetting, and incubating the mixture at 40°C for 1 hour to perform coupling reaction. Using a gel filtration spin column (BioRad), unreacted Cy5 was removed to purify each reaction solution, and nuclease-free water was added to the purified solution to attain a final volume of 32 $\mu$L. To the resulting solution, 8 $\mu$L of 5×Fragmentation Buffer, which is attached to the 3D-Gene Hybridization Buffer kit (Toray Industries, Inc.), was added, and the resulting mixture was lightly stirred by pipetting, followed by treatment at 94°C for 15 minutes. Each sample was rapidly cooled on crushed ice for 3 minutes and purified with Microcon YM-10 (Millipore).

(3) Microarray Analysis

Each aRNA after the labeling and purification was subjected to microarray analysis by the following operation. A solution containing 1000 ng of each RNA was prepared with nuclease-free water to a final volume of 16 $\mu$L, and 2 $\mu$L of Hybridization Buffer A in "3D-Gene" (registered trademark) Hybridization Buffer (Toray Industries, Inc.) was added thereto, followed by treating the resulting mixture at 95°C for 5 minutes. The mixture was rapidly cooled on crushed ice for 3 minutes, and 232 $\mu$L of Hybridization Buffer B was added thereto, followed by stirring the resulting mixture by gentle pipetting, thereby preparing 250 $\mu$L of a sample solution. The sample solution was degassed under reduced pressure, and 210 $\mu$L of the solution was applied to "3D-Gene", an entire mouse genomic DNA chip (Toray Industries, Inc.). Holes at 4 positions on the cover were closed by sealing, and the chip was placed in a hybridization chamber (Takara Bio Inc.,

TX711) immobilized on the top panel of Bioshaker (Tokyo Rikakikai, MMS-210). The temperature in the chamber was set to 37°C, and the sample was stirred with swirling rotation at 250 rpm, to allow hybridization reaction to proceed for 16 hours.

(4) Measurement of Fluorescent Signals

After the hybridization reaction, the cover member of the analysis chip was detached, and the substrate was washed and dried. The substrate was placed in a scanner (manufactured by Axon Instruments, GenePix 4000B) for DNA chips, and the signal value (fluorescence intensity) of each fluorescently labeled RNA subjected to the hybridization and the background noise were measured under the conditions of: laser output, 33%; and photomultiplier voltage setting, 500. Among all the spots, 1750 spots were provided as negative control spots for measurement of the background fluorescence value, and, from each signal value, the background signal value was subtracted, to calculate the true signal value for each spot. In case where the signal value was positive, the spot was defined as an "effective spot". The number of effective spots, as shown in Table 4, was: liver FFPE, 15571; cerebellum FFPE, 14897; shared effective spots of FFPE, 12597; liver frozen tissue, 17937; cerebellum frozen tissue, 19582; and shared effective spots of the frozen tissues, 16671. The cerebellum-liver signal ratio (cerebellum/liver) for each gene was determined with each of the FFPE samples (A) and the frozen tissues, and the data obtained from 11985 shared effective genes were plotted to prepare the scatter diagram shown in Fig. 3, taking the signal ratios for the frozen tissues along the ordinate and the signal ratios for the FFPE samples (A) along the abscissa.

The correlation coefficient R of the signal ratios of the above-described shared effective genes between the aRNAs obtained from RNA (A) derived from FFPE (A) and the control aRNAs obtained from RNA (D) derived from the frozen tissues was 0.905 (Table 4).

The correlation coefficient R herein is an index that quantitatively represents the intensity of the interrelationship between 2 data groups, and varies within the range between -1 and 1, wherein a positive value represents a positive correlation; a negative value represents a negative correlation, and the value zero represents no correlation. In general, in cases where the absolute value is not less than 0.5, it can be judged that there is a correlation; in cases where the absolute value is less than 0.5, it can be judged that there is no correlation; and the stronger the degree of correlation between 2 data groups, the closer the absolute value to 1. For calculation of the correlation coefficient with "Microsoft Office Excel" (Microsoft), the function "correl" may be used. In the present invention, in cases where genes whose expression was confirmed in common between the aRNA prepared from an RNA sample extracted from a fixed tissue or cell(s) and the control aRNA obtained from an RNA sample derived from a frozen tissue showed a correlation coefficient R of the signal ratios (cerebellum/liver) of not less than 0.7, the correlation was judged to be high. The correlation coefficient R is preferably not less than 0.8, more preferably not less than 0.9.

In the present Example 1, since the correlation between these was very high, it was revealed that, by the method of the present invention, aRNA less influenced by the amplification bias can be obtained even from a fixed FFPE sample, and, as a result, highly quantitative gene expression analysis is possible.

Example 2

In the same manner as in Example 1 except that the ratio of AA-UTP "molar amount of AA-UTP / (molar amount of UTP + molar amount of AA-UTP)" contained in the nucleotide reagent was set to 23 mol%, aRNA was prepared from 1 μg each of RNA (A) extracted from FFPE (A) (liver and cerebellum) and RNA (D) extracted from frozen tissues (liver and cerebellum). The yield of the obtained aRNA was: liver FFPE, 2.1 μg; cerebellum FFPE, 2.9 μg; liver frozen tissue, 8.5 μg; and cerebellum frozen tissue, 10.1 μg (the amplification factors were 2.1, 2.9, 8.5 and 10.1, respectively) (Table 4). As a result of confirmation of the sizes of the aRNAs in the same manner as in Example 1, the FFPE liver and cerebellum both showed a peak between 200 and 300 [nt], and the frozen liver and cerebellum tissues both showed a peak at about 500 [nt] (not shown).

Under the same conditions as in Example 1, microarray analysis was carried out to calculate the signal value of each gene. As shown in Table 4, the number of effective spots was: liver FFPE, 15910; cerebellum FFPE, 16229; shared effective spots of FFPE: 13391; liver frozen tissue, 18601; cerebellum frozen tissue, 20186; and shared effective spots of the frozen tissues, 17109. The correlation coefficient R of the signal ratios (cerebellum/liver) of the shared effective genes between the aRNA obtained from RNA (A) and the aRNA obtained from RNA (D) derived from the frozen tissues was 0.863, indicating a very high correlation between these (Table 4).

Example 3

In the same manner as in Example 1 except that RNA (A') was used instead of RNA (A) as an RNA sample and the ratio of AA-UTP "molar amount of AA-UTP / (molar amount of UTP + molar amount of AA-UTP)" contained in the nucleotide reagent was set to 10 mol%, aRNA was prepared from 250 ng each of RNA (A') extracted from FFPE (A) (liver and cerebellum) and (D) extracted from frozen tissues (liver and cerebellum). The yield of the obtained aRNA was: liver FFPE, 1.9 $\mu$g; cerebellum FFPE, 2.4 $\mu$g; liver frozen tissue, 3.5 $\mu$g; and cerebellum frozen tissue, 4.3 $\mu$g (the amplification factors were 7.6, 9.5, 14.1 and 17.3, respectively) (Table 4). As a result of confirmation of the sizes of the aRNAs in the same manner as in Example 1, the FFPE liver and cerebellum both showed a peak between 200 and 300 [nt], and the frozen liver and cerebellum tissues both showed a peak at about 500 [nt] (not shown).

Under the same conditions as in Example 1, microarray analysis was carried out to calculate the signal value of each gene. As shown in Table 4, the number of effective spots was: liver FFPE, 12385; cerebellum FFPE, 13960; shared effective spots of FFPE: 11401; liver frozen tissue, 16832; cerebellum frozen tissue, 17968; and shared effective spots of the frozen tissues, 15598. The correlation coefficient R of the signal ratios (cerebellum/liver) of the shared effective genes between the aRNA obtained from RNA (A') and the aRNA obtained from RNA (D) derived from the frozen tissues was 0.809, indicating a high correlation between these (Table 4).

[Table 4]

| Tissue sample | Example 1 | | | | Example 2 | | | | Example 3 | | | |
|---|---|---|---|---|---|---|---|---|---|---|---|---|
| | Frozen | | FFPE (A) | | Frozen | | FFPE (A) | | Frozen | | FFPE (A') | |
| | Cerebellum | Liver | Cerebellum | Liver | Cerebellum | Liver | Cerebellum | Liver | Cerebellum | Liver | Cerebellum | Liver |
| RNA sample | RNA (D) | | RNA (A) | | RNA (D) | | RNA (A) | | RNA (D) | | RNA (A') | |
| Initial RNA amount | 1 μg | | | | 1 μg | | | | 250 ng | | | |
| Number of amplification cycles | 1 | | | | 1 | | | | 1 | | | |
| AA-UTP/(UTP+AA-UTP) | 16% | | | | 23% | | | | 10% | | | |
| Amplification factor | 12.7 | 10.9 | 4.8 | 3.5 | 10.1 | 8.5 | 2.9 | 2.1 | 17.3 | 14.1 | 9.5 | 7.6 |
| Number of effective spots | 19582 | 17937 | 15571 | 14897 | 20186 | 18601 | 16229 | 15910 | 17968 | 16832 | 13960 | 12385 |
| Number of shared effective spots | 16671 | | 12597 | | 17109 | | 13391 | | 15598 | | 11401 | |
| Number of totally shared effective spots | 11985 | | | | 12198 | | | | 10291 | | | |
| Correlation coefficient between frozen and FFPE | 0.905 | | | | 0.863 | | | | 0.809 | | | |

Example 4

In the same manner as in Example 1 by setting the ratio of AA-UTP "molar amount of AA-UTP / (molar amount of UTP + molar amount of AA-UTP)" contained in the nucleotide reagent to 16 mol%, except that RNA (B) extracted from FFPE (B) (liver and cerebellum) was used instead of RNA (A) as an RNA sample, aRNA was prepared from 1 μg each of RNAs (B) and (D). The yield of the obtained aRNA was: liver FFPE, 2.9 μg; cerebellum FFPE, 3.9 μg; liver frozen tissue, 10.5 μg; and cerebellum frozen tissue, 12.6 μg (the amplification factors were 2.9, 3.9, 10.5 and 12.6, respectively) (Table 5). As a result of confirmation of the sizes of the aRNAs in the same manner as in Example 1, the FFPE liver and cerebellum both showed a peak between 200 and 300 [nt], and the frozen liver and cerebellum tissues both showed a peak at about 500 [nt] (not shown).
Under the same conditions as in Example 1, microarray analysis was carried out to calculate the signal value of each gene. As shown in Table 5, the number of effective spots was: liver FFPE, 15910; cerebellum FFPE, 16229; shared effective spots of FFPE: 13391; liver frozen tissue, 18601; cerebellum frozen tissue, 20186; and shared effective spots of the frozen tissues, 17109. The correlation coefficient R of the signal ratios (cerebellum/liver) of the shared effective genes between the aRNA obtained from RNA (B) and the aRNA obtained from RNA (D) derived from the frozen tissues was 0.863, indicating a very high correlation between these (Table 5).

Example 5

In the same manner as in Example 4 except that the ratio of AA-UTP "molar amount of AA-UTP / (molar amount of UTP + molar amount of AA-UTP)" contained in the nucleotide reagent was set to 23 mol%, aRNA was prepared from 1 μg each of RNA (B) extracted from FFPE (B) (liver and cerebellum) and RNA (D) extracted from frozen tissues (liver and cerebellum). The yield of the obtained aRNA was: liver FFPE, 2.0 μg; cerebellum FFPE, 2.7 μg; liver frozen tissue, 9.6 μg; and cerebellum frozen tissue, 10.5 μg (the amplification factors were 2.0, 2.7, 9.6 and 10.5, respectively) (Table 5). As a result of confirmation of the sizes of the aRNAs in the same manner as in Example 1, the FFPE liver and cerebellum both showed a peak between 200 and 300 [nt], and the frozen liver and cerebellum tissues both showed a peak at about 500 [nt] (not shown).
Under the same conditions as in Example 1, microarray analysis was carried out to calculate the signal value of each gene. As shown in Table 5, the number of effective spots was: liver FFPE, 14287; cerebellum FFPE, 15816; shared effective spots of FFPE: 12814; liver frozen tissue, 18821; cerebellum frozen tissue, 20105; and shared effective spots of the frozen tissues, 17501. The correlation coefficient R of the signal ratios (cerebellum/liver) of the shared effective genes between the aRNA obtained from RNA (B) and the aRNA obtained from RNA (D) derived from the frozen tissues was 0.851, indicating a very high correlation between these (Table 5).

Example 6

In the same manner as in Example 4 except that RNA (B') was used instead of RNA (B) as an RNA sample and the ratio of AA-UTP "molar amount of AA-UTP / (molar amount of UTP + molar amount of AA-UTP)" contained in the nucleotide reagent was set to 10 mol%, aRNA was prepared from 250 ng each of RNA (B') extracted from FFPE (B) (liver and cerebellum) and (D) extracted from frozen tissues (liver and cerebellum). The yield of the obtained aRNA was: liver FFPE, 1.6 μg; cerebellum FFPE, 2.0 μg; liver frozen tissue, 3.8 μg; and cerebellum frozen tissue, 4.2 μg (the amplification factors were 6.3, 8.1, 15 and 16.9, respectively) (Table 5). As a result of confirmation of the sizes of the aRNAs in the same manner as in Example 1, the FFPE liver and cerebellum both showed a peak between 200 and 300 [nt], and the frozen liver and cerebellum tissues both showed a peak at about 500 [nt] (not shown).
Under the same conditions as in Example 1, microarray analysis was carried out to calculate the signal value of each gene. As shown in Table 5, the number of effective spots was: liver FFPE, 11984; cerebellum FFPE, 13107; shared effective spots of FFPE: 10835; liver frozen tissue, 16914; cerebellum frozen tissue, 17819; and shared effective spots of the frozen tissues, 15681. The correlation coefficient R of the signal ratios (cerebellum/liver) of the shared effective genes between the aRNA obtained from RNA (B') and the aRNA obtained from RNA (D) derived from the frozen tissues was 0.803, indicating a high correlation between these (Table 5).

[Table 5]

| | Example 4 | | | | Example 5 | | | | Example 6 | | | |
|---|---|---|---|---|---|---|---|---|---|---|---|---|
| Tissue sample | Frozen | | FFPE (B) | | Frozen | | FFPE (B) | | Frozen | | FFPE (B') | |
| | Cerebellum | Liver | Cerebellum | Liver | Cerebellum | Liver | Cerebellum | Liver | Cerebellum | Liver | Cerebellum | Liver |
| RNA sample | RNA (D) | | RNA (B) | | RNA (D) | | RNA (B) | | RNA (D) | | RNA (B') | |
| Initial RNA amount | 1 μg | | | | 1 μg | | | | 250 ng | | | |
| Number of amplification cycles | 1 | | | | 1 | | | | 1 | | | |
| AA-UTP/(UTP+AA-UTP) | 16% | | | | 23% | | | | 10% | | | |
| Amplification factor | 12.6 | 10.5 | 3.9 | 2.9 | 10.5 | 9.6 | 2.7 | 2 | 16.9 | 15 | 8.1 | 6.3 |
| Effective spots | 19132 | 17828 | 14297 | 13588 | 20105 | 18821 | 15816 | 14287 | 17819 | 16914 | 13107 | 11984 |
| Shared effective spots | 16710 | | 11721 | | 17501 | | 12814 | | 15681 | | 10835 | |
| Totally shared effective spots | 10539 | | | | 11815 | | | | 9876 | | | |
| Correlation coefficient between frozen and FFPE | 0.886 | | | | 0.851 | | | | 0.803 | | | |

EP 2 484 760 B1

Example 7

In the same manner as in Example 1 by setting the ratio of AA-UTP "molar amount of AA-UTP / (molar amount of UTP + molar amount of AA-UTP)" contained in the nucleotide reagent to 16 mol%, except that RNA (C) extracted from FFPE (C) (liver and cerebellum) was used instead of RNA (A) as an RNA sample, aRNA was prepared from 1 μg each of RNAs (C) and (D). The yield of the obtained aRNA was: liver FFPE, 3.9 μg; cerebellum FFPE, 5.1 μg; liver frozen tissue, 10.3 μg; and cerebellum frozen tissue, 12.1 μg (the amplification factors were 3.9, 5.1, 10.3 and 12.1, respectively) (Table 6). As a result of confirmation of the sizes of the aRNAs in the same manner as in Example 1, the FFPE liver and cerebellum both showed a peak between 200 and 300 [nt], and the frozen liver and cerebellum tissues both showed a peak at about 500 [nt] (not shown).

Under the same conditions as in Example 1, microarray analysis was carried out to calculate the signal value of each gene. As shown in Table 6, the number of effective spots was: liver FFPE, 15328; cerebellum FFPE, 16540; shared effective spots of FFPE: 14309; liver frozen tissue, 17899; cerebellum frozen tissue, 19316; and shared effective spots of the frozen tissues, 16850. The correlation coefficient R of the signal ratios (cerebellum/liver) of the shared effective genes between the aRNA obtained from RNA (C) and the aRNA obtained from RNA (D) derived from the frozen tissues was 0.925, indicating a very high correlation between these (Table 6).

Example 8

In the same manner as in Example 7 except that the ratio of AA-UTP "molar amount of AA-UTP / (molar amount of UTP + molar amount of AA-UTP)" contained in the nucleotide reagent was set to 23 mol%, aRNA was prepared from 1 μg each of RNA (C) extracted from FFPE (C) (liver and cerebellum) and RNA (D) extracted from frozen tissues (liver and cerebellum). The yield of the obtained aRNA was: liver FFPE, 2.9 μg; cerebellum FFPE, 3.8 μg; liver frozen tissue, 10.3 μg; and cerebellum frozen tissue, 12.1 μg (the amplification factors were 2.9, 3.8, 10.3 and 12.1, respectively) (Table 6). As a result of confirmation of the sizes of the aRNAs in the same manner as in Example 1, the FFPE liver and cerebellum both showed a peak between 200 and 300 [nt], and the frozen liver and cerebellum tissues both showed a peak at about 500 [nt] (not shown).

Under the same conditions as in Example 1, microarray analysis was carried out to calculate the signal value of each gene. As shown in Table 6, the number of effective spots was: liver FFPE, 15944; cerebellum FFPE, 17027; shared effective spots of FFPE: 14895; liver frozen tissue, 18782; cerebellum frozen tissue, 20799; and shared effective spots of the frozen tissues, 17244. The correlation coefficient R of the signal ratios (cerebellum/liver) of the shared effective genes between the aRNA obtained from RNA (C) and the aRNA obtained from RNA (D) derived from the frozen tissues was 0.893, indicating a very high correlation between these (Table 6).

Example 9

In the same manner as in Example 7 except that RNA (C') was used instead of RNA (C) as an RNA sample and the ratio of AA-UTP "molar amount of AA-UTP / (molar amount of UTP + molar amount of AA-UTP)" contained in the nucleotide reagent was set to 10 mol%, aRNA was prepared from 250 ng each of RNA (C') extracted from FFPE (C) (liver and cerebellum) and (D) extracted from frozen tissues (liver and cerebellum). The yield of the obtained aRNA was: liver FFPE, 2.2 μg; cerebellum FFPE, 2.5 μg; liver frozen tissue, 3.7 μg; and cerebellum frozen tissue, 4.3 μg (the amplification factors were 8.5, 10.1, 14.8 and 17, respectively) (Table 6). As a result of confirmation of the sizes of the aRNAs in the same manner as in Example 1, the FFPE liver and cerebellum both showed a peak between 200 and 300 [nt], and the frozen liver and cerebellum tissues both showed a peak at about 500 [nt] (not shown).

Under the same conditions as in Example 1, microarray analysis was carried out to calculate the signal value of each gene. As shown in Table 6, the number of effective spots was: liver FFPE, 12543; cerebellum FFPE, 14139; shared effective spots of FFPE: 11734; liver frozen tissue, 16640; cerebellum frozen tissue, 17693; shared effective spots of the frozen tissues, 15342. The correlation coefficient R of the signal ratios (cerebellum/liver) of the shared effective genes between the aRNA obtained from RNA (C') and the aRNA obtained from RNA (D) derived from the frozen tissues was 0.861, indicating a high correlation between these (Table 6).

[Table 6]

| Tissue sample | Example 7 | | | | Example 8 | | | | Example 9 | | | |
|---|---|---|---|---|---|---|---|---|---|---|---|---|
| | Frozen | | FFPE (C) | | Frozen | | FFPE (C) | | Frozen | | FFPE (C') | |
| | Cerebellum | Liver | Cerebellum | Liver | Cerebellum | Liver | Cerebellum | Liver | Cerebellum | Liver | Cerebellum | Liver |
| RNA sample | RNA (D) | | RNA (C) | | RNA (D) | | RNA (C) | | RNA (D) | | RNA (C') | |
| Initial RNA amount | 1 µg | | | | 1 µg | | | | 250 ng | | | |
| Number of amplification cycles | 1 | | | | 1 | | | | 1 | | | |
| AA-UTP/(UTP+AA-UTP) | 16% | | | | 23% | | | | 10% | | | |
| Amplification factor | 12.1 | 10.3 | 5.1 | 3.9 | 10.6 | 8.3 | 3.8 | 2.9 | 17 | 14.8 | 10.1 | 8.5 |
| Effective spots | 19316 | 17899 | 16540 | 15328 | 20799 | 18782 | 17027 | 15944 | 17693 | 16640 | 14139 | 12543 |
| Shared effective spots | 16850 | | 14309 | | 17244 | | 14895 | | 15342 | | 11734 | |
| Totally shared effective spots | 13063 | | | | 13956 | | | | 10639 | | | |
| Correlation coefficient between frozen and FFPE | 0.925 | | | | 0.893 | | | | 0.861 | | | |

EP 2 484 760 B1

Comparative Example 1

In the same manner as in Example 1 except that the ratio of AA-UTP "molar amount of AA-UTP / (molar amount of UTP + molar amount of AA-UTP)" contained in the nucleotide reagent was set to 34 mol% and the amplification step of RNA was carried out twice, aRNA was prepared as follows from 1 μg each of RNA (A) extracted from FFPE (A) (liver and cerebellum) and RNA (D) extracted from frozen tissues (liver and cerebellum).

From 10 ng each of mouse cerebellum and liver RNAs (A), the first strand cDNA was synthesized using reverse transcriptase (SuperScript III (Invitrogen)), and DNA polymerase was then added thereto to synthesize the second strand cDNA which is complementary to the first strand DNA. The synthesized cDNA was purified through a silica gel-based column, and in vitro transcription reaction using T7 RNA polymerase was carried out at 42°C for 8 hours, to perform amplification reaction of aRNA. Using the thus obtained amplification product, first strand cDNA synthesis and second strand cDNA synthesis were carried out, and in vitro transcription reaction using a reagent containing AA-UTP was then carried out at 42°C for 10 hours, to perform amplification reaction of aRNA to which aminoallyl groups were introduced. The ratio of AA-UTP with respect to UTP + AA-UTP contained in the nucleotide reagent used in this in vitro transcription reaction "molar amount of AA-UTP / (molar amount of UTP + molar amount of AA-UTP)" was 34%. The amplified aRNA was purified using a silica gel-based column, and the yield was determined with a spectrophotometer (Nano Drop). As a result, the yield was: liver FFPE, 37.1 μg; cerebellum FFPE, 36.6 μg; liver frozen tissue, 53.5 μg; and cerebellum frozen tissue, 70.7 μg (the amplification factors were 3710, 3660, 5350 and 7070, respectively) (Table 7). As a result of confirmation of the sizes of the aRNAs in the same manner as in Example 1, the FFPE liver and cerebellum both showed a peak between 100 and 200 [nt], and the frozen liver and cerebellum tissues both showed a peak between 400 and 500 [nt] (not shown).

Under the same conditions as in Example 1, microarray analysis was carried out to calculate the signal value of each gene. As shown in Table 7, the number of effective spots was: liver FFPE, 13457; cerebellum FFPE, 14138; shared effective spots of FFPE: 10852; liver frozen tissue, 15824; cerebellum frozen tissue, 16699; and shared effective spots of the frozen tissues, 13647. The cerebellum-liver signal ratio (cerebellum/liver) for each gene was determined with each of the FFPE and frozen tissues, and the data obtained from 9556 shared effective genes were plotted to prepare the scatter diagram shown in Fig. 4, taking the signal ratios for the frozen tissues along the ordinate and the signal ratios for the FFPE samples (A) along the abscissa. The correlation coefficient R of the signal ratios (cerebellum/liver) of the shared effective genes between the aRNA obtained from RNA (A) and the aRNA obtained from RNA (D) derived from the frozen tissues was 0.495, indicating a low correlation between these (Table 7).

Comparative Example 2

In the same manner as in Example 1 except that the ratio of AA-UTP "molar amount of AA-UTP / (molar amount of UTP + molar amount of AA-UTP)" contained in the nucleotide reagent was set to 50 mol%, aRNA was prepared from 1 μg each of RNA (A) extracted from FFPE (A) (liver and cerebellum) and RNA (D) extracted from frozen tissues (liver and cerebellum). However, since a sufficient amount of aRNA could not be obtained with RNA (A) even by starting the amplification with 1 μg of the RNA, the amplification step was carried out after changing the RNA amount to 3 μg. The yield of the obtained aRNA was: liver FFPE, 3.6 μg; cerebellum FFPE, 5.1 μg; liver frozen tissue, 5.4 μg; and cerebellum frozen tissue, 7.7 μg (the amplification factors were 1.2, 1.7, 5.4 and 7.7, respectively) (Table 7). As a result of confirmation of the sizes of the aRNAs in the same manner as in Example 1, the FFPE liver and cerebellum both showed a peak between 100 and 200 [nt], and the frozen liver and cerebellum tissues both showed a peak between 400 and 500 [nt] (not shown).

Under the same conditions as in Example 1, microarray analysis was carried out to calculate the signal value of each gene. As shown in Table 7, the number of effective spots was: liver FFPE, 15091; cerebellum FFPE, 16209; shared effective spots of FFPE: 13622; liver frozen tissue, 18669; cerebellum frozen tissue, 20871; shared effective spots of the frozen tissues, 17049. The correlation coefficient R of the signal ratios (cerebellum/liver) of the shared effective genes between the aRNA obtained from RNA (A) and the aRNA obtained from RNA (D) derived from the frozen tissues was 0.687, indicating a low correlation between these (Table 7).

Comparative Example 3

In the same manner as in Example 1 except that the ratio of AA-UTP "molar amount of AA-UTP / (molar amount of UTP + molar amount of AA-UTP)" contained in the nucleotide reagent was set to 3 mol%, aRNA was prepared from 1 μg each of RNA (A) extracted from FFPE (A) (liver and cerebellum) and RNA (D) extracted from frozen tissues (liver and cerebellum). The yield of the obtained aRNA was: liver FFPE, 9.5 μg; cerebellum FFPE, 13.0 μg; liver frozen tissue, 18.8 μg; and cerebellum frozen tissue, 21.3 μg (the amplification factors were 9.5, 13, 18.8 and 21.3, respectively) (Table 7). As a result of confirmation of the sizes of the aRNAs in the same manner as in Example 1, the FFPE liver and cerebellum both showed a peak between 100 and 200 [nt], and the frozen liver and cerebellum tissues both showed a peak between 400 and 500 [nt] (not shown).

Under the same conditions as in Example 1, microarray analysis was carried out to calculate the signal value of each gene. As shown in Table 7, the number of effective spots was: liver FFPE, 6036; cerebellum FFPE, 7312; shared effective spots of FFPE: 5011; liver frozen tissue, 12789; cerebellum frozen tissue, 14871; and shared effective spots of the frozen tissues, 11049. It was thought that the number of effective spots was small in the present Reference Example because the amount of the aminoallyl groups introduced in the in vitro transcription reaction was small, and hence the level of labeling with a fluorescent label Cy5 was low, which resulted in a high background value. The correlation coefficient R of the signal ratios (cerebellum/liver) of the shared effective genes between the aRNA obtained from RNA (A) and the aRNA obtained from RNA (D) derived from the frozen tissues was 0.653, indicating a low correlation between these.

[Table 7]

| | Comparative Example 1 | | | | Comparative Example 2 | | | | Comparative Example 3 | | | |
|---|---|---|---|---|---|---|---|---|---|---|---|---|
| Tissue sample | Frozen | | FFPE (A) | | Frozen | | FFPE (A) | | Frozen | | FFPE (A) | |
| | Cerebellum | Liver | Cerebellum | Liver | Cerebellum | Liver | Cerebellum | Liver | Cerebellum | Liver | Cerebellum | Liver |
| RNA sample | RNA (D) | | RNA (A) | | RNA (D) | | RNA (A) | | RNA (D) | | RNA (A) | |
| Initial RNA amount | 10 ng | | | | 1 μg | | 3 μg | | 1 μg | | | |
| Number of amplification cycles | 2 | | | | 1 | | | | 1 | | | |
| AA-UTP/(UTP+AA-UTP) | 34% | | | | 50% | | | | 3% | | | |
| Amplification factor | 7070 | 5350 | 3660 | 3710 | 7.7 | 5.4 | 1.7 | 1.2 | 21.3 | 18.8 | 13 | 9.5 |
| Effective spots | 16699 | 15824 | 14138 | 13457 | 20871 | 18669 | 16209 | 15091 | 14871 | 12789 | 7312 | 6036 |
| Shared effective spots | 13647 | | 10852 | | 17049 | | 13622 | | 11049 | | 5011 | |
| Totally shared effective spots | 9556 | | | | 11964 | | | | 4128 | | | |
| Correlation coefficient between frozen and FFPE | 0.495 | | | | 0.687 | | | | 0.653 | | | |

INDUSTRIAL APPLICABILITY

In gene expression analysis using an RNA sample extracted from a fixed tissue or cell(s), the method of the present invention according to claim 1 for preparing aRNA provides a method for obtaining aRNA wherein the influence of the amplification bias is reduced. The present invention enables highly quantitative gene expression analysis and is especially effective in the field of medicine.

**Claims**

1.   A method for preparing amplified RNA (aRNA) to be used for gene expression analysis of an RNA sample wherein the RNA sample is extracted from a fixed tissue or cell(s)and the method comprises an RNA amplification step which comprises a reverse transcription step and an in vitro transcription step, wherein the ratio of 5-(3-aminoallyl)uridine 5'-triphosphate (AA-UTP) contained in a nucleotide reagent used in said in vitro transcription step is not less than 5 mol% and less than 25 mol% with respect to total of uridine 5'-triphosphate (UTP) and 5-(3-aminoallyl)uridine 5'-triphophate (AA-UTP) and wherein the number of times of the RNA amplification step to be carried out is one.

2.   The method for preparing amplified RNA according to claim 1, wherein said fixed tissue or cell(s) is/are embedded in paraffin.

3.   The method for preparing amplified RNA according to claim 1 or 2, wherein the average nucleotide length of said amplified RNA is not less than 200 nucleotides.

4.   Use of a nucleotide reagent for *in vitro* transcription in a method for preparing amplified RNA wherein the RNA sample is extracted from fixed tissue or cell(s) and wherein the nucleotide reagent contains a ratio of 5-(3-aminoallyl)uridine 5'-triphosphate with respect to total of uridine 5'-triphosphate and 5-(3-aminoallyl)uridine 5'-triphophate which is not less than 5 mol% and less than 25 mol%.

**Patentansprüche**

1.   Verfahren zur Herstellung von amplifizierter RNS (aRNS) zur Verwendung für eine Genexpressionsanalyse von einer RNS-Probe, wobei die RNS-Probe aus einem fixierten Gewebe oder fixierten Zelle(n) extrahiert wird und das Verfahren einen RNS-Amplifikationsschritt umfasst, der einen Reverse-Transkriptions-Schritt und einen In-Vitro-Transkriptionsschritt umfasst, wobei des Verhältnis von 5-(3-Aminoallyl)uridin-5'-triphosphat (AA-UTP), das in einem Nukleotidreagens, welches in dem In-Vitro-Transkriptionsschritt verwendet wird, enthalten ist, nicht weniger als 5 Mol-% und weniger als 25 Mol-% in Bezug auf die Gesamtheit an Uridin-5'-triphosphat (UTP) und 5-(3-Aminoallyl)uridin-5'-triphosphat (AA-UTP) ist und wobei die Anzahl, wie oft der RNS-Amplifikationsschritt durchgeführt wird, eins ist.

2.   Verfahren zur Herstellung von amplifizierter RNS nach Anspruch 1, wobei das fixierte Gewebe oder die fixierte(n) Zelle(n) in Paraffin eingebettet ist (sind).

3.   Verfahren zur Herstellung von amplifizierter RNS nach Anspruch 1 oder 2, wobei die durchschnittliche Nukleotidlänge der amplifizierten RNS nicht weniger als 200 Nukleotide ist.

4.   Verwendung eines Nukleotidreagens zur In-Vitro-Transkription in einem Verfahren zur Herstellung von amplifizierter RNS, wobei die RNS-Probe aus fixiertem Gewebe oder einer fixierten Zelle(n) extrahiert wird und wobei das Nukleotidreagens ein Verhältnis von 5-(3-Aminoallyl)uridin-5'-triphosphat von nicht weniger als 5 Mol-% und weniger als 25 Mol-% in Bezug auf die Gesamtheit an Uridin-5'-triphosphat und 5-(3-Aminoallyl)uridin-5'-triphosphat enthält.

**Revendications**

1.   Procédé de préparation d'ARN amplifié (ARNa) devant être utilisé pour une analyse d'expression génique d'un échantillon d'ARN, où l'échantillon d'ARN est extrait à partir d'un tissu ou d'une/de cellule(s) fixé(e)(s) et le procédé comprend une étape d'amplification d'ARN qui comprend une étape de transcription inverse et une étape de transcription *in vitro,* où le rapport de 5-(3-aminoallyl)uridine 5'-triphosphate (AA-UTP) contenu dans un réactif nucléotidique utilisé dans ladite étape de transcription *in vitro* n'est pas moins de 5 mol% et moins de 25 mol% par rapport au total d'uridine 5'-triphosphate (UTP) et de 5-(3-aminoallyl)uridine 5'-triphosphate (AA-UTP), et dans lequel le nombre de fois où l'étape d'amplification d'ARN doit être réalisée est une seule fois.

2. Procédé de préparation d'ARN amplifié selon la revendication 1, dans lequel ledit tissu ou ladite/lesdites cellule(s) fixé(e)(s) est/sont inclus(e)(s) en paraffine.

3. Procédé de préparation d'ARN amplifié selon la revendication 1 ou 2, dans lequel la longueur de nucléotides moyenne dudit ARN amplifié n'est pas moins de 200 nucléotides.

4. Utilisation d'un réactif nucléotidique pour réaliser une transcription *in vitro* dans un procédé de préparation d'ARN amplifié, où l'échantillon d'ARN est extrait à partir d'un tissu ou d'une/de cellule(s) fixé(e)(s) et où le réactif nucléotidique contient un rapport de 5-(3-aminoallyl)uridine 5'-triphosphate par rapport au total d'uridine 5'-triphosphate et de 5-(3-aminoallyl)uridine 5'-triphosphate qui n'est pas moins de 5 mol% et moins de 25 mol%.

[Figure 1]

(1) RNA(A)  (2) RNA(B)  (3) RNA(C)  (4) RNA(D)

[Figure 2]

(1) aRNA prepared from RNA(A)  (2) aRNA prepared from RNA(D)

[Figure 3]

[Figure 4]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009131262 A

**Non-patent literature cited in the description**

- **PETER A. C. 'T HOEN ; FLOOR DE KORT ; G. J. B. VAN OMMEN ; JOHAN T. DEN DUNNEN.** Fluorescent labelling of cRNA for microarray applications. *Nucleic Acids Research,* 2003, vol. 31 (5), e20